# EUROPÄISCHE PATENTSCHRIFT

(11) **EP 1 499 575 B1**
(45) Veröffentlichungstag und Bekanntmachung des Hinweises auf die Patenterteilung: **21.11.2007**
(21) Anmeldenummer: 03724978.6
(22) Anmeldetag: 09.04.2003
(51) Int. Cl.: C07C 43/23, C07C 41/26

(54) **VERFAHREN ZUR HERSTELLUNG VON ANISALKOHOL**
METHOD FOR PRODUCING ANIS ALCOHOL
PROCEDE DE PREPARATION D'ALCOOL ANISIQUE

(30) Priorität: 22.04.2002 DE 10217798
(43) Veröffentlichungstag der Anmeldung: 26.01.2005
(73) Patentinhaber: Symrise GmbH & Co. KG, 37603 Holzminden (DE)
(72) Erfinder: KUHN, Walter, 37603 Holzminden (DE); FUNK, Hans-Ulrich, 37697 Lauenförde (DE); SENFT, Gerhard, 37603 Holzminden (DE)
(74) Vertreter: Eisenführ, Speiser & Partner
(86) Internationale Anmeldenummer: PCT/EP2003/003647
(87) Internationale Veröffentlichungsnummer: WO 2003/089395

(56) Entgegenhaltungen:
- PATENT ABSTRACTS OF JAPAN vol. 1997, no. 01, 31. Januar 1997 (1997-01-31) & JP 08 238098 A (UNITIKA LTD), 17. September 1996 (1996-09-17)
- D.R. LEVERING ET.AL.: "The Promoter Effect of Platinic Choride on Raney Nickel. I. General Effects on Type W-6 Catalyst" J.AM.CHEM.SOC., Bd. 72, 1950, Seiten 1190-1194, XP002250853 in der Anmeldung erwähnt
- TAKAHIRO NISHIMURA ET.AL.: "Palladium(II)-Catalyzed Oxidation of Alkohols to Aldehydes and Ketones by Molecular Oxygen" J.ORG.CHEM., Bd. 64, 1999, Seiten 6750-6755, XP002250854

## Beschreibung

Die vorliegende Erfindung betrifft ein Verfahren zur Herstellung von Anisalkohol (Methoxybenzylalkohol) durch Hydrierung von Anisaldehyd (Methoxybenzaldehyd) in Gegenwart von Raney-Nickel und eines basischen Zusatzes, die Verwendung des Anisalkohols und Mittel enthaltend Anisalkohol.

p-Anisalkohol (4-Methoxybenzylalkohol) ist ein wichtiger und viel verwendeter Riechstoff mit süßem-blumigem, leicht balsamischem Geruch. Anisalkohol kommt beispielsweise in der Vanilleschote und dem Anissamen vor. (K. Bauer, A. Garbe, Common Fragrance and Flavor Materials, S. 89, VCH, Weinheim, 1985.; S. Arctander, Perfume and Flavour Chemicals, No. 249 1969).

In J. Am. Chem. Soc. 1959, 72, 1190 wird die Hydrierung von Anisaldehyd zu Anisalkohol mit Raney-Nickel unter Zugabe von Platin(IV)chlorid und Triethylamin beschrieben. Die Hydrierung wird in Ethanol als Lösungsmittel durchgeführt und ergibt Anisalkohol in 86 % Ausbeute.

In Bull. Chem. Soc. Jp. EN 3, 1983, 56, 719 wird die Hydrierung von Anisaldehyd zu Anisalkohol mit Raney-Nickel elektrokatalytisch beschrieben. Die Nickelkathode ist mit Raney-Nickel Pulver beschichtet und führt in methanolischer Lösung bei Raumtemperatur zu Anisalkohol in 71 % Ausbeute.

Die bekannten Verfahren sind zum einen durch die teuren, zum Teil auch aufwendig herstellbaren Katalysatoren nicht zufriedenstellend, zum anderen sind die Ausbeuten ungenügend.

Es bestand die Aufgabe, ein Verfahren zur Herstellung von Anisalkohol zu finden, das den Anisalkohol in besserer Ausbeute und in hoher Reinheit liefern kann.

Die vorliegende Erfindung betrifft daher ein Verfahren zur Herstellung von Anisalkohol durch Hydrierung von Anisaldehyd, dadurch gekennzeichnet, dass die Hydrierung in Gegenwart von Raney-Nickel und einem basischen Zusatz, gewählt aus der Reihe der Metallhydroxide oder der Metallalkoholate, durchgeführt wird.

Im Zuzammenhang mit der vorliegenden Erfindung stehen die Verwendung des erfindungsgemäß hergestellten Anisalkohols als Riechstoff sowie Mittel enthaltend diesen Anisalkohol, wie auch in JP08238098 offenbart.

Das erfindungsgemäße Verfahren ermöglicht insbesondere die Herstellung von sensorisch einwandfreiem Anisalkohol, insbesondere unter wirtschaftlichen Aspekten und im industriellen Maßstab.

Raney Nickel Katalysatoren sind an sich bekannt (Methoden der organischen Chemie/Houben Weyl, Band IV/1c, Reduktion Teil 1, Georg Thieme Verlag, Stuttgart, 1980, Seiten 15 bis 562).

In WO-A 2000/26165 ist die Hydrierung von Epoxiden in Gegenwart von Raney-Nickel und einer basischen Substanz beschrieben.

Für das erfindungsgemäße Verfahren können o-Anisaldehyd, p-Anisaldehyd, m-Anisaldehyd oder ein beliebiges Gemisch der genannten Aldehyde verwendet werden. Bevorzugt wird p-Anisaldehyd in die Hydrierung eingesetzt.

Für das erfindungsgemäße Verfahren kann das Raney-Nickel im trockenen oder feuchten Zustand (Wassergehalt bis zu 70 Gew.-%) verwendet werden.

Für das erfindungsgemäße Verfahren beträgt das Gewichtsverhältnis des eingesetzten Raney-Nickels zu Anisaldehyd 0,0001 bis 0,1 zu 1, bevorzugt 0,001. bis 0,05 zu 1, insbesondere bevorzugt 0,01 bis 0,02 zu 1.

Für das erfindungsgemäße Verfahren muss der basische Zusatz ein Metallhydroxid oder ein Metallalkoholat sein. Bevorzugt sind Alkalihydroxide und Erdalkalihydroxide sowie Alkalialkoholate und Erdalkalialkoholate, wobei die Alkoholate bevorzugt nicht mehr als 4 Kohlenstoffatome aufweisen. Besonders bevorzugte basische Zusätze sind Natriumhydroxid, Kaliumhydroxid, Natriummethylat, Natriumethylat.

Die basischen Zusätze können in reiner Form oder in Form von Lösungen eingesetzt werden. Bei den Hydroxiden eignen sich besonders wässrige Lösungen, bei den Alkoholaten eignen sich besonders alkoholische Lösungen.

Besonders bevorzugte Lösungen enthaltend basische Zusätze sind wässrige Natriumhydroxid-Lösung und methanolische Natriummethylat-Lösung.

Für das erfindungsgemäße Verfahren beträgt das Gewichtsverhältnis des eingesetzten basischen Zusatzes, bezogen auf dessen reine Form, zu Anisaldehyd 0,000001 bis 0,1 zu 1, bevorzugt 0,00001 bis 0,01 zu 1, insbesondere bevorzugt 0,0001 bis 0,001 zu 1.

Das Verfahren wird erfindungsgemäß bei 30-180°C, bevorzugt bei 70-130°C, insbesondere bevorzugt bei 80-100°C durchgeführt.

Das erfindungsgemäße Verfahren wird mit Wasserstoff durchgeführt, die Wasserstoffdrücke liegen üblicherweise im Bereich 1 bis 100 bar abs., bevorzugt ist eine Reaktionsführung bei Wasserstoffdrücken im Bereich 5 bis 50 bar abs., insbesondere im Bereich 10 bis 20 bar abs.

Die Reaktionszeit liegt üblicherweise im Bereich 2 bis 100 Stunden, bevorzugt im Bereich 5 bis 40 Stunden, insbesondere bevorzugt im Bereich 20 bis 25 Stunden.

Das Verfahren kann kontinuierlich, semi-kontinuierlich und diskontinuierlich durchgeführt werden.

Das erfindungsgemäße Verfahren kann unter Verwendung von Lösungsmitteln oder Lösungsmittelgemischen durchgeführt werden. Geeignet sind beispielsweise Alkohole, wässrige Alkohole, Ether, Ester, aromatische oder gesättigte Kohlenwasserstoffe. Üblicherweise können Lösungsmittel wie Methanol, Ethanol, Isopropanol, n-Propanol, Isobutanol, n-Butanol, sek.-Butanol, Tetrahydrofuran, Dibutylether, Ethylenglykoldimethylether, Ethylacetat, Methylacetat, Pentan, Hexan, Heptan, Octan, Cyclopentan, Cyclohexan, Methylcyclohexan, Cyclooctan, Benzol, Toluol, Ethylbenzol und Xylole verwendet werden.

Bevorzugt wird das Verfahren lösungsmittelfrei durchgeführt.

Das erfindungsgemäße Verfahren kann beispielsweise folgendermaßen durchgeführt werden:

In einem Druckbehälter werden Anisaldehyd, Raney-Nickel und basischer Zusatz vorgelegt. Es wird bei der gewählten Temperatur und dem gewähltem Wasserstoffdruck hydriert. Nach Ende der Hydrierung kann der rohe Anisalkohol durch Entfernen des Raney-Nickels (z.B. durch Filtration, Dekantierung, Zentrifugierung) und gegebenenfalls durch Entfernen des basischen Zusatzes über Waschvorgänge, erhalten werden. Bei Bedarf kann eine weitere Reinigung des Anisalkohols erfolgen, beispielsweise durch Destillation.

Nach dem erfindungsgemäßen Verfahren kann Anisalkohol in einer Reinheit von über 99 % und einer destillierten Ausbeute von 99 % erhalten werden.

Der nach dem erfindungsgemäßen Verfahren hergestellte Anisalkohol kann ohne weitere Aufreinigungsschritte eingesetzt werden, insbesondere weist er eine gute parfümistische Qualität auf.

Der nach dem erfindungsgemäßen Verfahren hergestellte Anisalkohol kann insbesondere verwendet werden als Riechstoff, in Riechstoffinischungen, Parfümkompositionen, Parfümölen oder Duftkompositionen.

Ein weiteres Verwendungsgebiet sind Hygiene- oder Pflegeprodukte, insbesondere im Bereich des Haushaltes und der Körperpflege.

Die den nach dem erfindungsgemäßen Verfahren hergestellten Anisalkohol enthaltenden Parfümöle können in konzentrierter Form, in Lösungen oder in sonstiger modifizierter Form verwendet werden für die Herstellung von z.B. Parfüm-Extraits, Eau de Parfums, Eau de Toilettes, Rasierwässer, Eau de Colognes, Pre-shave-Produkte, Splash-Colognes und parfümierten Erfrischungstüchern sowie die Parfümierung von sauren, alkalischen und neutralen Reinigungsmitteln, wie z.B. Fußbodenreinigern, Fensterglasreinigern, Geschirrspülmittel, Bad- und Sanitärreinigern, Scheuermilch, festen und flüssigen WC-Reinigem, pulver- und schaumförmigen Teppichreinigern, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ wie z.B. Hautcremes- und -lotionen, Gesichtscremes und -lotionen, Sonnenschutzcremes- und -lotionen, After-sun-cremes und -lotionen, Handcremes und -lotionen, Fußcremes und -lotionen, Enthaarungscremes und -lotionen, After-shave-Cremes und -lotionen, Bräunungscremes und -lotionen, Haarpflegeprodukten wie z.B. Haarsprays, Haargelen, Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z.B. Achselsprays, Roll-ons, Deosticks, Deocremes oder Produkten der dekorativen Kosmetik.

Folgendes Beispiel erläutert die Erfindung.

### Beispiel

In einen Rührautoklaven mit Begasungsrührer wurden 3000 g Anisaldehyd (GC-Reinheit 99,7 %.), 7,5 g 21 %ige Natriummethylatlösung in Methanol und 57 g feuchtes Raney-Nickel (50 % Wassergehalt) vorgelegt. Es wurde 21 Stunden bei 80 bis 100°C und 13 bar hydriert. Nach Filtration wurden 3015 g Anisalkohol mit einer Reinheit von 99,1 % erhalten. Der erhaltene Anisalkohol konnte bis 130°C Sumpftemperatur und 1 mbar Vakuum destilliert werden. Die Ausbeute d. Th. betrug 99 %.

## Patentansprüche

1. Verfahren zur Herstellung von Anisalkohol durch Hydrierung von Anisaldyhyd in Gegenwart von Raney-Nickel und einem basischen Zusatz, **dadurch gekennzeichnet, dass** die Hydrierung mit Wasserstoff erfolgt, der basische Zusatz gewählt wird aus der Reihe der Metallhydroxide oder der Metallakoholate, das Gewichtsverhältnis des eingesetzten Raney-Nickels zu Anisaldehyd 0,0001 bis 0,1:1 beträgt, das Gewichtsverhältnis des eingesetzten basischen Zusatzes, bezogen auf dessen reine Form, zu Anisaldehyd 0,000001 bis 0,0:1 beträgt und das Verfahren bei einer Temperatur von 30 bis 180 °C durchgeführt wird.

2. Verfahren nach Anspruch 1, **dadurch gekennzeichnet, dass** der basische Zusatz gewählt wird aus der Reihe Natriumhydroxid, Kaliumhydroxid, Natriummethylat oder Natriumethylat.

## Claims

1. Process for the production of anise alcohol by hydrogenating anisaldehyde in the presence of Raney nickel and a basic additive, **characterised in that** the hydrogenation is carried out with hydrogen, the basic additive is selected from the group comprising metal hydroxides or metal alcoholates, the weight ratio of the employed Raney nickel to anisaldehyde is 0.0001 to 0.1:1, the weight ratio of the employed basic additive, referred to its pure form, to anisaldehyde is 0.000001 to 0.0:1, and the process is carried out at a temperature of 30° to 180°C.

2. Process according to claim 1, **characterised in that** the basic additive is selected from the group comprising sodium hydroxide, potassium hydroxide, sodium methylate or sodium ethylate.

## Revendications

1. Procédé pour la préparation d'alcool anisique par hydrogénation d'aldéhyde anisique en présence de nickel de Raney et d'un additif basique, **caractérisé en ce que** l'on réalise l'hydrogénation avec de l'hydrogène, on choisit l'additif basique parmi les hydroxydes de métaux ou les alcoolates de métaux, le rapport massique du nickel de Raney utilisé à l'aldéhyde anisique est de 0,0001 à 0,1:1, le rapport massique de l'additif basique utilisé, rapporté à sa forme pure, par rapport à l'aldéhyde anisique est de 0,000001 à 0,1:1 et le procédé est réalisé à une température de 30 à 180°C.

2. Procédé selon la revendication 1, **caractérisé en ce que** l'on choisit l'additif basique parmi l'hydroxyde de sodium, l'hydroxyde de potassium, le méthylate de sodium ou l'éthylate de sodium.
